(19) **Europäisches Patentamt**

**European Patent Office**

**Office européen des brevets**

(11) **EP 0 916 625 B1**

(12) **EUROPÄISCHE PATENTSCHRIFT**

(45) Veröffentlichungstag und Bekanntmachung des Hinweises auf die Patenterteilung:
**18.09.2002  Patentblatt 2002/38**

(51) Int Cl.⁷: **C03C 10/00**, A61K 6/06, C03C 10/04

(21) Anmeldenummer: **98250392.2**

(22) Anmeldetag: **09.11.1998**

(54) **Verfahren zur Herstellung von geformten transluzenten Lithiumdisilikat-Glaskeramik-Produkten**

Process to prepare moulded shaped translucent lithium-di-silicate glass ceramic products

Procédé de production de produits moulés en vitrocéramique translucide de disilicate de lithium

(84) Benannte Vertragsstaaten:
**AT CH DE FR GB IT LI SE**

(30) Priorität: **10.11.1997  DE 19750794**

(43) Veröffentlichungstag der Anmeldung:
**19.05.1999  Patentblatt 1999/20**

(73) Patentinhaber: **Ivoclar Vivadent AG**
**9494 Schaan (LI)**

(72) Erfinder:
- **Schweiger, Marcel, Dipl.-Ing.**
 **7000 Chur (CH)**
- **Cramer von Clausbruch, Sascha**
 **6830 Rankweil (AT)**
- **Höland, Wolfram, Prof. Dr.**
 **9494 Schaan (LI)**
- **Rheinberger, Volker, Dr.**
 **9490 Vaduz (LI)**

(74) Vertreter: **UEXKÜLL & STOLBERG**
**Patentanwälte**
**Beselerstrasse 4**
**22607 Hamburg (DE)**

(56) Entgegenhaltungen:
**EP-A- 0 690 031**         **EP-A- 0 827 941**
**EP-A- 0 885 606**         **US-A- 4 189 325**
**US-A- 4 515 634**

Bemerkungen:
Die Akte enthält technische Angaben, die nach dem Eingang der Anmeldung eingereicht wurden und die nicht in dieser Patentschrift enthalten sind.

## Beschreibung

Die Erfindung betrifft ein Verfahren zur Herstellung von geformten transluzenten Lithiumdisilikat-Glaskeramik-Produkten, welche als Rohlinge herstellbar sind, die insbesondere durch plastische Verformung unter Druck- und Wärmeeinwirkung oder spanende Bearbeitung zu geformten transluzenten Dentalprodukten mit hoher Festigkeit verarbeitet werden können.

Lithiumdisilikat-Glaskeramiken sind aus dem Stand der Technik bekannt. So werden in der EP-B-536 479 selbstglasierte Lithiumdisilikat-Glaskeramik-Gegenstände beschrieben, die jedoch nicht für dentale Zwecke vorgesehen sind. Die Glaskeramiken enthalten zudem kein $La_2O_3$, und es wird ebenfalls nicht beschrieben, Rohlinge aus der Glaskeramik herzustellen, die nach Bearbeitung einer weiteren Wärmebehandlung zur Vervollständigung der Kristallisation unterworfen werden. Auch ist es erforderlich, die Wärmebehandlung bei sehr geringer Aufheizrate von 5K/min durchzuführen, um Spannungen im Gefüge der Glaskeramik zu vermeiden.

Weiter ist die Glaskeramik primär zur Herstellung von Tafelgeschirr vorgesehen, welches naturgemäß eine nur geringe Transluzenz hat.

Auch die EP-B-536 572 beschreibt Lithiumdisilikat-Glaskeramiken, die kein $La_2O_3$ enthalten. Durch Aufstreuen eines feinteiligen gefärbten Glases auf ihre Oberfläche erhalten sie Struktur und Färbung, und sie werden als Auskleidungselemente für Bauzwecke eingesetzt.

In der US-A-4,189,325 werden Lithiumdisilikat-Glaskeramiken offenbart, die zwingend Calciumoxid zur Fließverbesserung sowie als besondere Keimbildner Platin und Nioboxid zur Erzeugung von sehr feinen und einheitlichen Kristallen enthalten. Auch wenn die Glaskeramik in Form von noch nicht vollständig kristallisierten Rohlingen erzeugt werden kann, so ist sie dennoch frei von $La_2O_3$.

Die WO-A-95/32678 und US-A-5,507,981 beschreiben Lithiumdisilikat-Glaskeramiken, die bei Einsatz eines speziellen verpreßbaren Tiegels durch Heißpressen zu geformten Dentalprodukten verarbeitet werden können. Allerdings werden die glaskeramischen Materialien soweit erhitzt, daß keine Kristalle mehr im geschmolzenen Material vorliegen, da sonst die Viskosität für das Verpressen zu dem Dentalprodukt zu hoch ist. Untersuchungen haben gezeigt, daß beim Verpressen der beschriebenen Materialien mittels des in der EP-A-231 773 beschriebenen Verfahrens sowie unter Verwendung des dort offenbarten Preßofens eine unerwünscht starke Reaktion mit der eingesetzten Einbettmasse auftritt. Außerdem zeigen die eingesetzten Gläser eine sehr hohe Kristallwachstumsgeschwindigkeit, so daß bei der Wärmebehandlung große Kristalle entstehen, die das Gefüge der erzeugten Glaskeramik stören und demgemäß zu Produkten mit nur geringer Fertigkeit führen.

Die EP-A-827 941 offenbart sinterbare Lithiumdisilikat-Glaskeramiken, aus denen geformte Dentalprodukte durch Verpressen im viskosen Zustand erzeugt werden können. Zur Herstellung der glaskeramischen Dentalprodukte wird das Pulver eines entsprechenden Ausgangsglases zu einem Ausgangsglas-Rohling gewünschter Geometrie und heterogener Struktur dichtgepresst, und dieser Rohling wird anschließend einer oder mehreren Wärmebehandlungen unterworfen, um das glaskeramische Dentalprodukt zu ergeben.

Weiter sind aus der DE-C-1 421 886 Glaskeramiken auf der Basis von $SiO_2$ und $Li_2O$ bekannt, welche große Mengen an physiologisch sehr bedenklichem Arsenoxid enthalten.

In der US-A-4,515,634 wird eine zur Herstellung von Dentalkronen und -brücken geeignete Lithiumdisilikat-Glaskeramik offenbart, die jedoch keinerlei $La_2O_3$ enthält.

Auch die in FR-A-2 655 264 beschriebenen Glaskeramiken sind frei von $La_2O_3$. Sie enthalten Lithiumoxid und Siliciumoxid sowie sehr große Mengen an MgO und sind zur Herstellung von Dentalprothesen geeignet.

Weiter sind aus dem Stand der Technik auch Rohlinge aus Sinterkeramik auf Basis von Leucit, Feldspat oder Glimmer bekannt, die mittels computer-gestützter Fräsverfahren zu Dentalprodukten verarbeitet werden. Diese Produkte besitzen allerdings nur geringe Festigkeit, weshalb sich diese Materialien für hochbelastete Dentalrestaurationen nicht durchgesetzt haben.

Die bekannten Lithiumdisilikat-Glaskeramiken zeigen Unzulänglichkeiten bei ihrer Weiterverarbeitung zu geformten Produkten, da bei ihrer Verarbeitung im plastischen Zustand unter Einsatz von erhöhten Temperaturen und erhöhten Drucken eine unerwünscht starke Reaktion mit der bei dem Verpressen eingesetzten Einbettmasse auftritt. Eine Weiterverarbeitung der Glaskeramiken durch spanende Bearbeitung, wie Fräsen, ist infolge der Festigkeit und Zähigkeit der Glaskeramiken in der Regel nicht in zufriedenstellendem Maße durchführbar. Weiter zeigen die herkömmlichen Lithiumdisilikat-Glaskeramiken häufig nicht die für Dentalprodukte erforderlichen hohen Festigkeiten und optischen Eigenschaften, wie hohe Transluzenz, und ihnen mangelt es in vielen Fällen auch an der für den Einsatz als Dentalmaterial erforderlichen chemischen Stabilität, welches in der Mundhöhle permanent mit Fluiden unterschiedlichster Art umspült wird.

Der Erfindung liegt demgemäß die Aufgabe zugrunde, ein Verfahren zur Herstellung von geformten transluzenten Lithiumdisilikat-Glaskeramik-Produkten zur Verfügung zu stellen, die eine hohe chemische Stabilität, eine geringe Fehlstellendichte sowie eine hohe Transluzenz bei gleichzeitig guten mechanischen Eigenschaften haben und bei Weiterverarbeitung durch Verpressen im plastischen Zustand eine nur geringe Reaktion mit der eingesetzten Einbettmasse zeigen, und die Glaskeramik-Produkte auch in Form von Rohlingen mit geringem Kristallisationsgrad hergestellt werden können, die in einfacher Weise durch maschinelle Bearbeitung, wie spanende Verfahren, in gewünschter Weise geformt und durch anschließende Wärmebehandlung zu einem hochfesten Glaskeramik-Produkt umgewandelt werden können.

Diese Aufgabe wird durch das Verfahren zur Herstellung von geformten transluzenten Lithiumdisilikat-Glaskeramik-Produkten nach den Ansprüchen 1 bis 12 gelöst.

Gegenstand der Erfindung sind ebenfalls die geformten Glaskeramik-Produkte nach den Ansprüchen 13 bis 15, die Verwendung nach Anspruch 16 sowie die geformten Dentalprodukte nach den Ansprüchen 17 bis 19.

Das erfindungsgemäße Verfahren zur Herstellung von geformten transluzenten Lithiumdisilikat-Glaskeramik-Produkten zeichnet sich dadurch aus, daß man

(a) eine Schmelze eines Ausgangsglas erzeugt, welches die folgenden Komponenten enthält,

| Komponente | Gew.-% |
|---|---|
| $SiO_2$ | 57,0 bis 80,0 |
| $Al_2O_3$ | 0 bis 5,0 |
| $La_2O_3$ | 0,1 bis 6,0 |
| MgO | 0 bis 5,0 |
| ZnO | 0 bis 8,0 |
| $Li_2O$ | 11,0 bis 19,0 |

wobei

(i) $Al_2O_3 + La_2O_3$     0,1 bis 7,0 Gew.-% und
(ii) MgO + ZnO     0,1 bis 9,0 Gew.-%

ausmachen,

(b) die Schmelze des Ausgangsglases in der gewünschten Weise formt und abkühlt, und

(c) das geformte Glas-Produkt mindestens einer Wärmebehandlung im Temperaturbereich von 400 bis 1100°C unterzieht, um ein als Rohling vorliegendes geformtes Glaskeramik-Produkt zu erhalten.

In Verfahrensschritt (a) wird eine Schmelze eines Ausgangsglases erzeugt, wozu geeignete Ausgangsmaterialien, wie z.B. Carbonate, Oxide, Phosphate und Fluoride, innig miteinander vermischt und auf Temperaturen von insbesondere 1200 bis 1600 °C erwärmt werden. Zur Erzielung einer besonders hohen Homogenität kann die erzeugte Glasschmelze unter Bildung eines Glasgranulates in Wasser eingegossen und das erhaltene Glasgranulat erneut bei Temperaturen von insbesondere 1200 bis 1600 °C während 1 bis 4 Stunden erschmolzen werden.

Die Schmelze der Ausgangsglases enthält vorzugsweise mindestens eine der folgenden weiteren Komponenten:

| Komponente | Gew.-% |
|---|---|
| $ZrO_2$ | 0 bis 10,0 |
| $K_2O$ | 0 bis 13,5 |
| $P_2O_5$ | 0 bis 11,0 |
| Farb- und Fluoreszenzkomponenten | 0 bis 8,0 |
| Zusatzkomponenten | 0 bis 6,0 |

Erstaunlicherweise wurde festgestellt, daß der zusätzliche Einbau von $ZrO_2$ zu einer Erhöhung der Transluzenz führte, obwohl der gegenteilige Effekt in der konventionellen Glaskeramik gemäß EP-B-536 479 beobachtet wurde.

Für die Mengen der einzelnen Komponenten existieren, sofern nicht anders angegeben, unabhängig voneinander wählbare Bereiche, die wie folgt sind:

| Komponente | Gew.-% | |
|---|---|---|
| $SiO_2$ | 57,0 bis 75,0 | |
| $Al_2O_3$ | 0 bis 2,5 | |
| $La_2O_3$ | 0,1 bis 4,0 | |
| MgO | 0,1 bis 5,0 | |
| ZnO | 0 bis 6,0, | insbesondere 0,1 bis 5,0 |
| $ZrO_2$ | 0 bis 8,0, | insbesondere 0,1 bis 8,0 |
| $K_2O$ | 0 bis 9,0, | insbesondere 0,5 bis 7,0 |
| $Li_2O$ | 13,0 bis 19,0 | |
| $P_2O_5$ | 0 bis 8,0, | insbesondere 0,5 bis 8,0 |
| Farb- und Fluoreszenzkomponenten | 0,1 bis 8,0 | |
| Zusatzkomponenten | 0 bis 3,0. | |

Als Farbkomponente oder Fluoreszenzkomponente können z.B. Oxide von f-Elementen verwendet werden. Bevorzugt wird mindestens eine der folgenden Verbindungen eingesetzt.

| Komponente | Gew.-% |
|---|---|
| $CeO_2$ | 0,1 bis 5,0 |
| $V_2O_5$ | 0,01 bis 1,0 |
| $Fe_2O_3$ | 0,01 bis 1,0 |
| $MnO_2$ | 0,01 bis 3,0 |
| $TiO_2$ | 0,01 bis 5,0 |
| $Y_2O_3$ | 0,01 bis 2,0 |
| $Er_2O_3$ | 0,001 bis 2,0 |
| $Tb_2O_3$ | 0,001 bis 2,0 |
| $Eu_2O_3$ | 0,001 bis 2,0 |
| $Yb_2O_3$ | 0,001 bis 2,0 |
| $Gd_2O_3$ | 0,001 bis 2,0 |
| $Nd_2O_3$ | 0,001 bis 2,0 |
| $Pr_2O_3$ | 0,001 bis 2,0 |
| $Dy_2O_3$ | 0,001 bis 2,0 |
| $Ag_2O$ | 0,01 bis 2,0 |
| $SnO_2$ | 0,01 bis 3,0 |
| $Ta_2O_5$ | 0,001 bis 2,0 |

Die speziellen im erfindungsgemäßen Verfahren als Farb- oder Fluoreszenzkomponente einsetzbaren Oxide gewährleisten eine einfache Anpassung der Farbe des Glaskeramik-Produktes an den jeweiligen Anwendungszweck. Dies ist von besonderer Bedeutung, wenn die Glaskeramik-Produkte als Dentalprodukte eingesetzt werden sollen, deren Farbe an die des natürlichen Zahnmaterials des jeweiligen Patienten speziell angepaßt werden muß. Das mit diesen speziellen Oxiden erzielbare Farbspektrum reicht von sehr hellen Tönen bis zu stark grau-braunen Tönen z.B. bei devitalen Zahnstümpfen. Dabei wird durch etwaige vorhandene Fluoreszenzkomponenten die Fluoreszenz des natürlichen Zahnmaterials imitiert. Ein besonderer Vorteil der erfindungsgemäß eingesetzten Farb- und Fluoreszenzkomponenten ist, daß sie das Gefüge der erzeugten Glaskeramik-Produkte nicht in der Weise stören, daß inhomogene Materialien mit hoher Fehlstellendichte und hoher Porosität erzeugt werden. Dieses Problem tritt häufig bei Sinterkeramiken auf, deren Farbe durch Zugabe von Pigmenten verändert wird. Zur Vermeidung von Einbußen ihrer färbenden Wirkung werden diese Pigmente in der Regel erst vor Durchführung des bei relativ niedrigen Temperaturen durchgeführten Sintervorganges zugegeben, so daß sie stets als Kristalle oder Kristallite vorliegen, die zu Inhomogenitäten führen.

Neben den zuvor erwähnten Komponenten kann das Ausgangsglas auch noch Zusatzkomponenten enthalten, wofür insbesondere $B_2O_3$, $Na_2O$, BaO, F und/oder SrO in Frage kommen.

Bevorzugt besteht die Schmelze des Ausgangsglases aus den erwähnten Komponenten in den angegebenen Mengen.

Weiter wird in Stufe (b) die Schmelze des Ausgangsglases in der gewünschten Weise geformt und abgekühlt. Dabei erfolgt das Formen insbesondere durch Eingießen der Schmelze in eine gewünschte Form. Es ist weiter möglich, daß nach dem Eingießen auch noch eine Verdichtung der Schmelze durch Druck erfolgt, um eine besonders gute Homogenität und genaue Abbildung zu erzielen. Dabei kann in der Weise vorgegangen werden, daß ein Glastropfen in die gewünschte Form eingebracht und anschließend durch Pressen verdichtet wird.

Die Abkühlung der Schmelze erfolgt insbesondere in kontrollierter Weise, um damit den mit schnellen Temperaturwechseln einhergehenden Spannungen im Gefüge und daraus möglicherweise resultierenden Rissen und Sprüngen vorzubeugen. In der Regel wird daher die Schmelze in vorgewärmte Formen eingegossen oder in einem Ofen langsam abgekühlt.

Schließlich wird in Stufe (c) das gebildete geformte Glas-Produkt mindestens einer Wärmebehandlung unterzogen, um auf diese Weise dessen Kristallisation zu bewirken. Nach Abschluß dieser Verfahrensstufe wird ein als Rohling vorliegendes geformtes Glaskeramik-Produkt erhalten. Dieser Rohling hat üblicherweise die Form eines kleinen Zylinders oder eines rechteckigen Blockes. Die Wärmebehandlung erfolgt vorzugsweise bei einer Temperatur von weniger als 1000 und insbesondere weniger als 900 °C. Dabei wird das geformte Glasprodukt vorzugsweise in einen bereits auf die genannte Temperatur aufgeheizten Ofen eingebracht. Im Gegensatz zu konventionellen Materialien ist es nicht erforderlich, eine langsame Aufheizgeschwindigkeit zu wählen, um Spannungen auszuschließen. Für dieses vorteilhafte Verhalten ist offenbar die spezielle Zusammensetzung und Herstellungsweise des erfindungsgemäßen Materials verantwortlich.

Der Kristallisationsgrad und die Kristallgröße in diesem Glaskeramik-Rohling kann durch die Art der gewählten Wärmebehandlung in einem sehr breiten Bereich variiert werden. So ist es einerseits möglich, ein Glas mit lediglich Keimen oder sehr kleinen Kristallen im Submikron-Bereich zu erzeugen, was somit die einfachste Form einer Glaskeramik darstellt, oder andererseits eine vollständig kristallisierte Glaskeramik zu bilden. In jedem Fall läuft der Keramisierungsprozeß über den Mechanismus der Volumenkristallisation ab, und für die Bildung von im Gefüge feinverteilten Kristallen spielen in dem eingesetzten Ausgangsglas vorhandene Volumenkeimbildner, wie z.B. $P_2O_5$, eine wesentliche Rolle.

Zur Erzeugung des endgültigen Glaskeramik-Produktes, wie z.B. einer dentalen Brücke oder einer dentalen Krone, bestehen insbesondere die folgenden zwei Möglichkeiten (d1) und (d2).

Zum einen kann in Stufe (d1) das als Rohling vorliegende Glaskeramik-Produkt bei einer Temperatur von 700 bis 1200 °C und durch Anwendung von Druck, insbesondere von 8 bis 40 bar, zu einem Glaskeramik-Produkt gewünschter Geometrie plastisch verformt werden. Für diesen Formgebungsschritt ist es bevorzugt, das in der EP-A-231 773 beschriebene Verfahren zur Herstellung von Zahnersatzteilen sowie den dort ebenfalls offenbarten Preßofen zu benutzen. Bei diesem Verfahren wird der Rohling im plastischen Zustand in einen dem gewünschten geformten Dentalprodukt, wie z.B. Kronen, entsprechenden Formhohlraum unter Anwendung von Wärme und Druck eingepreßt. Der hierfür insbesondere eingesetzte Preßofen wird als Empress®-Ofen von der Ivoclar AG, Liechtenstein, vertrieben.

Es hat sich gezeigt, daß herkömmliche Lithiumdisilikat-Glaskeramiken bei der Weiterverarbeitung zu Glaskeramik-Produkten durch plastische Verformung eine unangemessen hohe Reaktion mit der verwendeten Einbettmasse zeigen, in nur ungenügendem Maße fließen oder unkontrolliertes Kristallwachstum zeigen. Diese Nachteile werden bei dem erfindungsgemäßen Verfahren durch die Verwendung von $La_2O_3$ und gegebenenfalls $Al_2O_3$ in den angegebenen Mengen im Ausgangsglas vermieden. Hierdurch kann das als Rohling vorliegende Glaskeramik-Produkt durch das Verpressen im plastischen Zustand in vorteilhafter Weise zu einem Glaskeramik-Produkt gewünschter Geometrie, insbesondere einem Dentalprodukt, wie einer Dentalrestauration, verarbeitet werden.

Weiter ist es möglich, das als Rohling vorliegende Glaskeramik-Produkt in Stufe (d2) durch spanende Bearbeitung, insbesondere CAD/CAM-gestützte Fräsgeräte, zu einem Glaskeramik-Produkt gewünschter Geometrie zu verarbeiten. Damit ist eine sogenannte "chair-side" Behandlung für den Zahnarzt möglich. Bei Durchführung dieser Weiterverarbeitungsvariante wird insbesondere ein Glaskeramik-Rohling eingesetzt, der noch nicht vollständig kristallisiert ist, sondern z.B. lediglich als keimhaltiger Glas-Rohling oder Glaskeramikrohling mit sehr kleinen Kristallen vorliegt. Derartige noch nicht vollständig kristallisierte Glaskeramik-Rohlinge zeigen den besonderen Vorteil, daß sie in deutlicher einfacherer Weise als herkömmliche Glaskeramiken maschinell zu dem fertigen Glaskeramik-Produkt gewünschter Geometrie verarbeitet werden können. Zur Herstellung eines Glaskeramik-Rohlings, bei dem die Glasmatrix lediglich Keime oder sehr kleine Kristallite enthält, hat es sich als besonders vorteilhaft erwiesen, die in Stufe (c) durchgeführte Wärmebehandlung bei einer Temperatur von 400 bis 900 °C durchzuführen. In jedem Fall kann der Kristallinitätsgrad des eingesetzten Glaskeramik-Rohlings an die Art der gewünschten spanenden Bearbeitung angepaßt werden, um diese möglichst einfach durchführen zu können.

Nach der anschließenden spanenden Bearbeitung in Stufe (d2) wird das erhaltene geformte Glaskeramik-Produkt dann mindestens einer weiteren Wärmebehandlung, insbesondere bei 700 bis 900 °C, unterzogen, um eine weitere Kristallisation und damit Verfestigung des Glaskeramik-Produktes zu erzielen. Durch diese weitere Wärmebehandlung werden die Bruchfestigkeit, die Farbe und die Transluzenz verbessert.

Das nach der Weiterverarbeitung, insbesondere in den Stufen (d1) und (d2), vorliegende fertige Glaskeramik-Produkt gewünschter Geometrie kann schließlich noch mit einer Beschichtung versehen werden, was gerade bei dessen Einsatz im Dentalbereich vorteilhaft ist. Als Beschichtung kommen dabei insbesondere eine Keramik, eine Sinterkeramik, eine Glaskeramik, bevorzugt eine Apatit-Glaskeramik, ein Glas, eine Glasur und/oder ein Composit in Frage. Vorteilhaft sind solche Beschichtungen, die eine Sintertemperatur von 650 bis 950 °C und einen linearen thermischen Ausdehnungskoeffizient haben, welcher kleiner ist als der des zu beschichtenden Glaskeramik-Produktes. Besonders vorteilhaft sind Beschichtungen, deren linearer thermischer Ausdehnungskoeffizient nicht mehr als $\pm\,3{,}0 \times 10^{-6}$ K$^{-1}$ von dem des Substrats abweicht.

Das Aufbringen einer Beschichtung erfolgt insbesondere durch Aufsinterung. Während dieses Sintervorganges wird das die Lithiumdisilikat-Glaskeramik enthaltende Glaskeramik-Produkt allerdings in einen Temperaturbereich gebracht, der oberhalb des Transformationspunktes der Restglasmatrix der Glaskeramik liegt. Herkömmliche Lithiumdisilikat-Glaskeramiken werden dabei häufig in unerwünschter Weise deformiert, da sie eine zu geringe Temperaturstandfestigkeit haben. Das erfindungsgemäß hergestellte Glaskeramik-Produkt hat jedoch eine ausgezeichnete Temperaturstandfestigkeit, wofür insbesondere der Gehalt an La$_2$O$_3$ und gegebenenfalls Al$_2$O$_3$ in den angegebenen Mengen verantwortlich ist.

Die erfindungsgemäß hergestellten Glaskeramik-Produkte sind aufgrund ihrer Eigenschaften besonders zum Einsatz als Dentalprodukte oder Bestandteile von diesen geeignet. So haben bevorzugte Glaskeramik-Produkte eine 3-Punkt-Biegefestigkeit von mehr als 400 MPa; wenn sie nach Verfahrensvariante (d1) hergestellt werden, und mehr als 250 MPa, wenn sie nach Verfahrensvariante (d2) hergestellt werden. Das zur Ermittlung der 3-Punkt-Biegefestigkeit benutzte Verfahren ist in den Beispielen erläutert.

Weiter haben die erfindungsgemäßen Glaskeramik-Produkte eine mit der des natürlichen Zahnes vergleichbare Transluzenz. Zur Quantifizierung der Transluzenz wurde nach dem in den Beispielen beschriebenen Meßverfahren der CR-Wert bestimmt. Der CR-Wert, auch als Kontrastverhältnis bezeichnet, gibt das Verhältnis der Lichtreflexion eines Probekörpers der Glaskeramik auf schwarzem Hintergrund zu der Messung der Lichtreflexion eines Probekörpers auf weißem Hintergrund an und dient somit als Maß für die Transluzenz eines Materials. Der CR-Wert ist durch die folgende Formel definiert:

$$CR = Y_b/Y_w$$

mit

CR =   Kontrastverhältnis,
$Y_b$ =   Lichtreflexion des Probekörpers auf schwarzem Hintergrund, und
$Y_w$ =   Lichtreflexion des Probekörpers auf weißem Hintergrund.

Der CR-Wert liegt stets im Bereich von 0 bis 1, wobei CR = für eine Opazität von 0 % und demnach ein vollständiges transluzentes Material und CR = 1 für eine Opazität von 100 % und demnach ein vollständig opakes, d. h. lichtundurchlässiges Material steht.

Das erfindungsgemäße Glaskeramik-Produkt hat üblicherweise einen CR-Wert von 0,05 bis 0,9 und bevorzugt von 0,1 bis 0,75, jeweils gmessen bei einer Probendicke von 1,2 mm.

Auch haben Untersuchungen des erfindungsgemäßen Glaskeramik-Produktes gezeigt, daß dieses ein sehr homogenes Gefüge mit gleichmäßig verteilten feinen Kristallen aufweist. Es wird angenommen, daß dieses spezielle Gefüge durch die besondere Zusammensetzung des eingesetzten Ausgangsglases sowie das Formen, insbesondere Gießen von Massivglas-Rohlingen in Stufe (b) hervorgerufen wird und für die besonders hohe Festigkeit des schließlich erhaltenen Glaskeramik-Produktes verantwortlich ist.

Es ist zudem überraschend, daß die Farbe, Transluzenz und Fluoreszenz des erfindungsgemäßen Glaskeramik-Produktes an die eines natürlichen Zahnes angepaßt werden kann, ohne daß die dabei eingesetzten Farb- und Fluoreszenzkomponenten die Festigkeit und Zähigkeit der Glaskeramik nachteilig beeinflussen würden. Im Gegensatz dazu ist bei Glaskeramiken auf Leucit-Basis bekannt, daß durch solche Zusätze die Kristallisation stark beeinflußt und die Festigkeit häufig sehr verringert wird. So ist es bei Sinterkeramiken bekannt, daß die in ihnen vielfach eingesetzten Pigmente zu einer recht hohen Fehlstellendichte und einer Porenbildung in der Glaskeramik führen, was wiederum deren Eigenschaften verschlechtert.

Schließlich zeichnet sich das erfindungsgemäße Glaskeramik-Produkt durch eine ausgezeichnete Säurebeständigkeit aus, die vorzugsweise weniger als 100 µg/cm$^2$ Masseverlust beträgt. Dieser Masseverlust wurde nach dem in den Beispielen erläuterten Verfahren bestimmt, bei dem die Glaskeramik über einen bestimmten Zeitraum mit wäßriger Essigsäure behandelt wird, und der nach der Behandlung festgestellte Masseverlust als Maß für die Säurebeständigkeit dient.

Bevorzugte geformte Dentalprodukte, die das erfindungsgemäße Glaskeramik-Produkt aufweisen, sind Dentalrestaurationen, wie z.B. ein Inlay, ein Onlay, eine Brücke, ein Stiftaufbau, eine Verblendung, ein Veneer, eine Facette, eine Krone oder eine Teilkrone.

Weiter sind solche geformten Dentalprodukte bevorzugt, die als Rohlinge vorliegen, d.h. bei denen noch eine Weiterverarbeitung zu dem endgültigen Dentalprodukt, z.B. nach den Stufen (d1) und (d2) erfolgt. Solche Rohlinge können in verschiedenen dem jeweiligen Weiterverarbeitungsverfahren angepaßten Formen vorliegen, wie z.B. kleine Zylinder oder rechteckige Blöcke.

Die Erfindung wird im folgenden anhand von Beispielen näher erläutert.

### Beispiele

### Beispiele 1 bis 20

Es wurden insgesamt 20 verschiedene erfindungsgemäße Glaskeramik-Produkte mit der in Tabelle I angegebenen chemischen Zusammensetzungen hergestellt, indem die Stufen (a) bis (c) des beschriebenen Verfahrens durchgeführt wurden.

Tabelle I (Angaben in Gew.-%)

| Bei-spiel | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
| $SiO_2$ | 67,9 | 66,6 | 69,42 | 68,86 | 68,5 | 68,1 | 64,9 | 68,1 | 67,6 | 74,95 | 61 | 71,7 | 67,6 | 66,5 | 67,9 | 61,3 | 63,7 | 65,6 | 66,7 | 65,8 |
| $K_2O$ | 4,2 | 4,1 | 4,3 | 4,3 | 4,2 | 4,2 | 5,2 | 4,2 | 4,1 | 2 | 7,8 | 4,4 | 4,1 | 3,5 | 4,2 | 13,5 | 4 | 4,1 | 4,1 | 4,1 |
| $Li_2O$ | 15 | 14,7 | 15,4 | 15,3 | 15,1 | 15,1 | 16,1 | 15 | 15 | 17 | 11 | 15,9 | 14,9 | 15,6 | 15,1 | 13,8 | 14 | 14,5 | 14,8 | 14,6 |
| $Al_2O_3$ | 1,1 | 1 | 1,1 | 1,1 | 1,1 | 1,1 | 1,1 | 1,1 | 1,1 | 0,5 | 2 | | 1,1 | 1,5 | 1,1 | 1 | 1 | 1,1 | 1,1 | 1,1 |
| $P_2O_5$ | 3,8 | 3,7 | 3,8 | 3,8 | 3,8 | 3,8 | 3,7 | 3,8 | 3,8 | 1,8 | 7 | | 3,8 | 2,5 | 3,7 | 3,4 | 3,6 | 3,7 | 3,7 | 3,7 |
| $MgO$ | 0,1 | 0,1 | 0,1 | 0,1 | 0,1 | 0,1 | 0,1 | 0,1 | 0,1 | 0,05 | 0,2 | 0,1 | 0,1 | 0,1 | 0,1 | 0,1 | 0,1 | 0,1 | 0,1 | 0,1 |
| $TiO_2$ | 1,6 | 1,6 | | 0,3 | | | | | | | | | | 0,2 | | | | 0,2 | | |
| $ZrO_2$ | | 2 | | | | | | | | | | | | | | | 6,1 | 4 | | 3 |
| $ZnO$ | 4,8 | 4,7 | 4,9 | 4,9 | 4,8 | 4,8 | 5,8 | 4,8 | 4,8 | 2,3 | 8 | 5,1 | 4,8 | 5 | 4,8 | 4,1 | 4,6 | 4,7 | 4,7 | 4,7 |
| $CeO_2$ | 0,5 | 0,5 | 0,6 | 0,6 | 2 | 2 | 2 | 2 | 2 | 1 | 2 | 2 | 2 | 2 | 2 | 2 | 2 | 0,5 | 2 | |
| $MnO_2$ | 0,53 | 0,53 | | 0,32 | | | | | | | | | | 1,8 | | | | | | |
| $Fe_2O_3$ | 0,17 | 0,17 | | 0,12 | | | | | | | | | | | | | | | | |
| $La_2O_3$ | 0,3 | 0,3 | 0,3 | 0,3 | 0,3 | 0,3 | 0,3 | 0,3 | 0,3 | 0,15 | 0,5 | 0,3 | 0,3 | 0,3 | 0,3 | 0,3 | 0,3 | 1 | 0,3 | 2 |
| $Ag_2O$ | | | 0,08 | | | | | | | | | | | | | | | | | |
| $V_2O_5$ | | | | | 0,1 | 0,1 | 0,2 | 0,6 | 0,6 | 0,1 | 0,2 | 0,2 | 0,1 | 0,2 | 0,2 | 0,2 | | | 0,2 | |
| $Er_2O_3$ | | | | | | 0,1 | 0,3 | | | | | | 0,1 | | | | | | | |
| $Tb_4O_7$ | | | | | | 0,3 | | | 0,4 | | | | | 1,1 | | | | | | |
| $Eu_2O_3$ | | | | | | | 0,3 | | | | | | | | | | | | | |

| | | | | | | | | | | | |
|---|---|---|---|---|---|---|---|---|---|---|---|
| Pr$_2$O$_3$ | | 0,3 | | 0,6 | 0,3 | 0,6 | 0,3 | 0,3 | 0,3 | 0,15 | |
| Y$_2$O$_3$ | 0,6 | | | | | | 0,5 | | | | 0,2 |
| Dy$_2$O$_3$ | | | 0,5 | | | | | | | | |
| SnO$_2$ | | 2 | | | | | | | | | |
| Ta$_2$O$_5$ | 0,3 | | | | | | | | | | |

Beispiel 21

Dentalprodukt hergestellt durch Heißpressen gemäß EP-A-231 773

Dieses Beispiel beschreibt die Herstellung eines erfindungsgemäßen Glaskeramik-Rohlings, welcher zur Herstellung eines individuell formbaren vollkeramischen Dentalproduktes, wie z.B. eine Krone oder eine mehrgliedrige Brücke, dienen kann. Auf das Dentalprodukt kann dann zusätzlich eine angepaßte Dentalsinterkeramik aufgebrannt werden.

Zunächst wurde ein Ausgangsglas mit der in der Tabelle I für Beispiel 14 angegebenen chemischen Zusammensetzung hergestellt. Dazu wurde ein Gemenge aus entsprechenden Oxiden, Carbonaten und Phosphaten in einer Kugelmühle gemischt und in einem Platin/-Rhodium-Tiegel bei einer Temperatur von 1500 °C und einer Homogenisierungszeit von 2 Stunden erschmolzen. Die erhaltene Glasschmelze wurde durch Eingießen in Wasser granuliert, und die gebildete Glasfritte wurde getrocknet. Dann wurde die Glasfritte erneut in eine Kugelmühle aufgemahlen und nochmals bei 1500 °C und einer Homogenisierungszeit von 2 Stunden geschmolzen. Die erhaltene homogene, transparente und leicht gelb gefärbte Schmelze wurde anschließend in eine bei 500 °C vorgewärmte Stahlform zu zylindrischen Stäben gegossen, die in einem Ofen kontrolliert von 500 °C langsam auf Raumtemperatur abgekühlt wurden. Die erhaltenen Glasstäbe wurden zu Proben von 2 g, 3 g und 4 g gesägt und dann 30 Minuten lang bei 870 °C wärmebehandelt, um die entsprechenden Glaskeramik-Rohlinge zu bilden. Dabei wurden die abgekühlten Glasstäbe direkt in den auf 870 °C vorgeheizten Ofen gelegt. Eine langsame Aufheizgeschwindigkeit war nicht erforderlich.

Rohlingeigenschaften

Die erhaltenen Glaskeramik-Rohlinge wiesen vergleichbare optische Eigenschaften, wie z.B. Transluzenz, Färbung und Trübung, wie übliche dentalkeramische Verkaufsprodukte auf, z.B. IPS Empress-Rohlinge von Ivoclar AG, Liechtenstein.

A. 3-Punkt-Biegefestigkeit

Zur Bestimmung der 3-Punkt-Biegefestigkeit wurden aus den Glaskeramik-Rohlingen Stäbe als Prüfkörper gemäß ISO Dentalnorm 6872/1995 gefertigt. Die Ermittlung der 3-Punkt-Biegefestigkeit erfolgte dann ebenfalls gemäß ISO 6872-1995 E "Dental Ceramic" mit einer Vorschubgeschwindigkeit der Lastaufbringung von 0,5 mm/min und einer Stützweite des Prüfmittels von 15 mm. Die unter diesen Bedingungen ermittelte Biegefestigkeit betrug 408 ± 63 MPa.

Eigenschaften plastisch verformter Glaskeramik

Die erhaltenen Glaskeramik-Rohlinge wurden unter Verwendung des Heißpreßverfahrens gemäß EP-A-231 773 und des dort ebenfalls beschriebenen Preßofens unter Vakuum im viskosen Zustand in die für die jeweiligen Test gewünschte Probengeometrie verpreßt. Dabei betrugen die Bereitschaftstemperatur des Preßofens 800 °C, die Heizrate bis zur Preßtemperatur 60 °C/min, die Preßtemperatur 910 °C, die Haltezeit bei der Preßtemperatur 15 Minuten und die Preßdruckanzeige 5 bar. Nach dem Preßvorgang wurde die Preßform an der Luft abgekühlt, und die erhaltenen geformten Glaskeramik-Produkte wurden durch Sandstrahlen mit $Al_2O_3$-Pulver und Glasperlen entformt. Die Produkte hatten folgende Eigenschaften.

A. Optische Eigenschaften

Zur Quantifizierung der Transluzenz der Glaskeramik-Produkte wurde der CR-Wert nach dem Meßverfahren der British Standards Institution ermittelt, welches in der Prüfnorm von Dentalkeramik "BS 5612: 1978" beschrieben ist.

Hierzu wurden 5 Prüfkörper mit einem Durchmesser von 16 mm und einer Probendicke von 1,4 mm hergestellt. Die Prüfkörper wurden mit nassem SiC-Pulver, Körnung 320, beschliffen, um die gewünschte Oberflächengüte zu erhalten (Oberflächenrauhigkeit Ra = 0,8 μm bis 1,6 μm). Dabei ist es wichtig, daß die Planparallelität der gegenüberliegenden Seiten eine Toleranz von ± 0,01 mm nicht überschreitet, da das Meßergebnis in hohem Maße von der Schichtstärke abhängt. Die endgültige Probenhöhe/-dicke betrug 1,2 ± 0,025 mm.

Die Prüfkörper wurden in einem Farbmeßinstrument Minolta-CR300 in die vorgesehene Meßöffnung gelegt, und die Remission von jedem der 5 Prüfkörper wurde mit einer Blende von 10 mm gemessen. Bei der Messung dürfen die Proben nicht in optischen Kontakt mit dem Hintergrund sein, was gegebenenfalls durch Aufgabe eines Tropfens Glycerin auf den Hintergrund verhindert wurde.

(a) Zur Ermittlung der Probenemission auf schwarzem Hintergrund $Y_b(Y_{black})$ wurde ein schwarzes Plättchen mit nicht mehr als 4 % Reflexion benutzt.

(b) Zur Ermittlung der Probenemission auf weißem Hintergrund $Y_w(Y_{white})$ wurde ein weißes Plättchen mit einer Reflexion von 80 % bis 85 % benutzt.

Aus den ermittelten Werten $Y_b$ und $Y_w$ wurde dann der Kontrastwert $CR = Y_b/Y_w$ berechnet, und er betrug 0,63.

Infolge der transluzenten Eigenschaften war dieses Glaskeramik-Produkt als vollkeramisches Dentalprodukt geeignet, das optisch den Vorgaben eines natürlichen Zahnes entspricht. Durch den Einsatz von glasfärbenden Oxiden im Ausgangsglas war das heißgepreßte Glaskeramik-Produkt zahnfarben getönt, wobei die Farbintensität und -tönung durch die Konzentration der färbenden Oxide eingestellt werden konnte.

Auf das als Gerüstmaterial einsetzbare transluzente Glaskeramik-Produkt konnte eine transluzente bis transparente Dentalsinterglaskeramik mit einem Ausdehnungskoeffizienten von $9,5 \times 10^{-6}$ K$^{-1}$ (100 bis 400 °C) als Beschichtung aufgebracht werden. Dabei wurde die Dentalsinterglaskeramik schichtweise auf das Glaskeramik-Produkt unter Vakuum bei 760 °C aufgesintert, was zu transluzenten vollkeramischen Dentalrestaurationen führte, die den hohen ästhetischen Anforderungen an derartige Produkte genügen.

### B. 3-Punkt-Biegefestigkeit

Die 3-Punkt-Biegefestigkeit wurde an heißgepreßten Glaskeramik-Stäben entsprechend dem oben für die Rohlinge benutzten Verfahren bestimmt. Dabei wurde eine 3-Punkt-Biegefestigkeit von $450 \pm 85$ MPa ermittelt.

### C. Linearer thermischer Ausdehnungskoeffizient

Hierfür wurden zylindrische Glaskeramik-Proben mit einem Durchmesser von 6 mm und einer Länge von 20 mm heißgepreßt. Der im Temperaturbereich von 100 bis 500 °C für diese Proben bestimmte Ausdehnungskoeffizient betrug $10,8 \times 10^{-6}$ K$^{-1}$.

### D. Bruchzähigkeit $K_{IC}$

Hierzu wurden Glaskeramik-Stäbe mit den Maßen $20 \times 4,4 \times 1,4$ mm$^3$ heißgepreßt und anschließend mit SiC-Naßschleifpapier (1000er Körnung) allseitig überschliffen. Mit einer Diamanttrennscheibe (0,2 mm Dicke) wurden die Proben einseitig bis zur Mitte auf eine Tiefe von 2,2 mm gekerbt und anschließend nach DIN 51 109 bei einer äußeren Stützweite von 15 mm und einer Vorschubgeschwindigkeit der Lastaufbringung von 0,5 mm/min mittels 4-Punkt-Biegeanordnung geprüft. Der ermittelte $K_{IC}$-Wert betrug $3,0 \pm 0,3$ MPa $\sqrt{m}$.

### E. Säurebeständigkeit

Hierzu wurden scheibenförmige Glaskeramik-Proben mit einem Durchmesser von 15 mm und einer Dicke von 1,5 mm heißgepreßt und anschließend mit SiC-Naßschleifpapier (1000er Körnung) allseitig überschliffen. Der gemäß ISO 6872-1995 E "Dental Ceramic" bestimmte flächenbezogene Masseverlust dieser Proben wurde nach 16-stündiger Lagerung in 4 vol.-%iger wäßriger Essigsäurelösung bestimmt. Der Wert betrug 36 $\mu$g/cm$^2$ und lag deutlich unter dem Normwert für Dentalkeramiken von 2000 $\mu$g/cm$^2$.

### Beispiel 22

### Dentalprodukt hergestellt durch computergesteuerte Frästechnik

Dieses Beispiel beschreibt die Herstellung eines erfindungsgemäßen Glaskeramik-Rohlings, der durch spanende Bearbeitung und anschließend erneute Wärmebehandlung zu einem individuell geformten vollkeramischen Dentalprodukt, wie z.B. eine Krone oder eine mehrgliedrige Brücke, verarbeitet wird, auf das zusätzlich eine angepaßte transluzente bis transparente Dentalsinterkeramik aufgebrannt werden kann.

Die Herstellung des Dentalproduktes erfolgte über ein CAD/CAM-Verfahren, wie z.B. CEREC 2®, von Siemens AG.

Erst die nach der spanenden Bearbeitung durchgeführte Wärmebehandlung ergab das Dentalprodukt mit den hohen mechanischen Kennwerten, wie 3-Punkt-Biegefestigkeit, und den für ein dentalkeramisches Produkt geforderten guten optischen Eigenschaften.

Zunächst wurde ein Ausgangsglas mit der in der Tabelle I für Beispiel 7 angegebenen Zusammensetzung hergestellt. Dazu wurde ein Gemenge aus Oxiden, Carbonaten und Phosphaten in einer Kugelmühle gemischt und in einem Platin/Rhodium-Tiegel bei einer Temperatur von 1500 °C und einer Homogenisierungszeit von 2 Stunden erschmolzen. Die Glasschmelze wurde durch Eingießen in Wasser gefrittet, und die Fritte wurde nach Trocknung in einer Kugelmühle aufgemahlen und nochmals bei 1500 °C und einer Homogenisierungszeit von 2 Stunden geschmolzen. Die erhaltene homogene, transparente und leicht gelb gefärbte Schmelze wurde dann in eine auf 500 °C vorgewärmte Stahlform zu rechteckigen Blöcken mit den Dimensionen $65 \times 20 \times 16$ mm$^3$ gegossen und in einem Temperofen kontrolliert von 500 °C langsam auf Raumtemperatur abgekühlt. Die erhaltenen rechteckigen Glasblöcke wurden zu Proben mit den Maßen $18 \times 14 \times 20$ mm$^3$ gesägt. Diese Proben wurden dann 60 Minuten bei 650 °C wärmebehandelt. Dabei wurden die abgekühlen Glasblöcke direkt in den auf 650 °C aufgeheizten Ofen eingebracht. Die nach diesem ersten Temperschritt erhaltenen Glaskeramik-Rohlinge hatten die folgenden Eigenschaften.

Rohlingeigenschaften nach Einfachtemperung (650 °C/1h)

A. Optische Eigenschaften

Die Glaskeramik-Rohlinge besaßen eine violett-weißliche Farbe. Sie hatten einen nach dem in Beispiel 21 beschriebenen Verfahren bestimmten CR-Wert von 0,36.

B. 3-Punkt-Biegefestigkeit

Die gemäß Beispiel 21 für die Glaskeramik-Rohlinge bestimmte 3-Punkt-Biegefestigkeit betrug $171 \pm 20$ MPa.

Herstellung und Eigenschaften fertiger Glaskeramik

Die einfach wärmebehandelten Glaskeramik-Rohlinge wurden mittels einer computergesteuerten Fräsmaschine zu dentalkeramischen Restaurationen, wie z.B. Kronen, verarbeitet. Aufgrund der relativ geringen Festigkeit und Zähigkeit der Glaskeramik-Rohlinge erwies sich die Verarbeitung als einfach durchführbar. Im Vergleich zu bekannten Fräskeramiken riefen sie einen geringeren Werkzeugverschleiß hervor, und es bildeten sich weniger Ausbrüche, was auf die feinere Struktur und Fehlstellenfreiheit zurückführbar ist.

Die gefräste dentale Restauration wurde dann einer weiteren Wärmebehandlung bei 760 °C während 1 h unterzogen. Diese Temperatur wurde gewählt, da bei ihr keine Gefahr einer Verformung des Gerüsts besteht. Diese zusätzliche Wärmebehandlung führte zu einer weitergehenden Kristallisation und damit Änderung der Eigenschaften der Restauration. Die erhaltene zweifach behandelte Glaskeramik hatte folgende Eigenschaften.

A. Optische Eigenschaften

Die Glaskeramik war transluzent und durch den Einsatz von glasfärbenden Oxiden im Ausgangsglas zahnfarben getönt.

Der für diese Glaskeramik gemäß Beispiel 21 bestimmte CR-Wert an zylindrischen Proben mit einem Durchmesser von 16 mm und einer Dicke von 1,2 mm betrug 0,23.

B. 3-Punkt-Biegefestigkeit

Die gemäß Beispiel 21 bestimmte 3-Punkt-Biegefestigkeit der Glaskeramik betrug $272 \pm 24$ MPa.

C. Linearer thermischer Ausdehnungskoeffizient

Hierzu wurden rechteckige Prüfkörper mit den Maßen $30 \times 4 \times 3$ mm$^3$ aus zweifach wärmebehandelten Massivblöcken gesägt. Der im Temperaturbereich von 100 bis 500 °C für diese Proben bestimmte Ausdehnungskoeffizient betrug $10,9 \times 10^{-6}$ K$^{-1}$.

D. Bruchzähigkeit $K_{IC}$

Die gemäß Beispiel 21 an Stäben aus zweifach thermischen behandelten Massivblöcken ermittelte Bruchzähigkeit betrug $2,1 \pm 0,1$ MPa $\sqrt{m}$.

E. Säurebeständigkeit

Die gemäß Beispiel 21 an Proben von zweifach wärmebehandelter Glaskeramik bestimmte Säurebeständig-

keit betrug 16 $\mu$g/cm$^2$ und war damit deutlich unter dem Normwert für Dentalkeramikmaterialien von 2000 $\mu$g/cm$^2$ und niedriger als der von konventionellen dentalen Gerüstmaterialien.

Auf die gefräste und zweifach wärmebehandelte Glaskeramik wurde schließlich eine transluzente bis transparente Sinterglaskeramik mit einem Ausdehnungskoeffizienten von 9,5•10$^{-6}\times$K$^{-1}$ schichtweise bei 760 °C und einer jeweiligen Haltezeit von 2 Minuten unter Vakuum aufgesintert. Dadurch entstand eine fertige dentale Restauration.

### Beispiele 23 bis 26

In diesen Beispielen wurden heißgepreßte Glaskeramik-Produkte entsprechend Beispiel 21 hergestellt und auf ihre Eigenschaften überprüft. Als Ausgangsgläser wurden jedoch Gläser mit der in Tabelle I für Beispiele 1, 4, 18 und 20 angegebenen Zusammensetzung verwendet.

Die für diese Glaskeramiken sowie die Glaskeramiken gemäß Beispiel 21 und 22 bestimmten Eigenschaften sind in Tabelle II aufgeführt.

Tabelle II

| Beispiele (Ausgangs-glas) | Verfahren | Biegefestigkeit [MPa] | Bruchzähig-keit [MPa*√ m] | Thermischer Ausdehnungs-koeffizient (100 °C - 500 °C) [μm/mK] | Säurebeständigkeit [μg/cm²] | Fluoreszenz (bei 366 nm Wellenlänge) | Transluzenz CR-Wert (Probendicke 1,2 mm) | Farbe |
|---|---|---|---|---|---|---|---|---|
| Beispiel 21 (Glas Nr. 14) | d1 | 450 ± 85 | 3,0 ± 0,3 | 10,8 | 36 | weiß-gelb | 0,63 | weiß-beige |
| Beispiel 22 (Glas Nr. 7) | d2 | 272 ± 24 | 2,1 ± 0,1 | 10,9 | 16 | orange | 0,23 | grau-beige |
| Beispiel 23 (Glas Nr. 1) | d1 | 386 ± 71 | | 10.7 | 22 | | | weiß-beige |
| Beispiel 24 (Glas Nr. 4) | d1 | 453 ± 93 | | 10,5 | 34 | | | weiß-gelb |
| Beispiel 25 (Glas Nr. 18) | d1 | 336 ± 63 | | | | weiß-gelb | 0,41 | gelblich-transparent |
| Beispiel 26 (Glas Nr. 20) | d1 | 343 ± 15 | | | | dunkel-violett | 0,37 | weißlich-transparent |

14

**Patentansprüche**

1. Verfahren zur Herstellung von geformten transluzenten Lithiumdisilikat-Glaskeramik-Produkten, indem man

   (a) eine Schmelze eines Ausgangsglas erzeugt, welches die folgenden Komponenten enthält

   | Komponente | Gew.-% |
   |------------|--------|
   | $SiO_2$ | 57,0 bis 80,0 |
   | $Al_2O_3$ | 0 bis 5,0 |
   | $La_2O_3$ | 0,1 bis 6,0 |
   | $MgO$ | 0 bis 5,0 |
   | $ZnO$ | 0 bis 8,0 |
   | $Li_2O$ | 11,0 bis 19,0 |

   wobei

   (i) $Al_2O_3$ + $La_2O_3$    0,1 bis 7,0 Gew.-% und
   (ii) $MgO$ + $ZnO$    0,1 bis 9,0 Gew.-%

   ausmachen,

   (b) die Schmelze des Ausgangsglases in der gewünschten Weise formt und abkühlt, und

   (c) das geformte Glas-Produkt mindestens einer Wärmebehandlung im Temperaturbereich von 400 bis 1100°C unterzieht, um ein als Rohling vorliegendes geformtes Glaskeramik-Produkt zu erhalten.

2. Verfahren nach Anspruch 1, **dadurch gekennzeichnet, daß** man

   (d1)    das als Rohling vorliegende Glaskeramik-Produkt bei einer Temperatur von 700 bis 1200 °C und durch Anwendung von Druck, insbesondere von 8 bis 40 bar, zu einem Glaskeramik-Produkt gewünschter Geometrie plastisch verformt.

3. Verfahren nach Anspruch 1, **dadurch gekennzeichnet, daß** man

   (d2)    das als Rohling vorliegende Glaskeramik-Produkt durch spanende Bearbeitung zu einem Glaskeramik-Produkt gewünschter Geometrie verarbeitet.

4. Verfahren nach Anspruch 3, **dadurch gekennzeichnet, daß** man in (d2) einen Rohling einsetzt, der einer Wärmebehandlung bei 400 bis 900°C unterzogen worden ist.

5. Verfahren nach Anspruch 3 oder 4, **dadurch gekennzeichnet, daß** man das in Stufe (d2) erhaltene geformte Glaskeramik-Produkt gewünschter Geometrie mindestens einer weiteren Wärmebehandlung, insbesondere bei 700 bis 900°C unterzieht.

6. Verfahren nach einem der Ansprüche 1 bis 5, **dadurch gekennzeichnet, daß** man die Wärmebehandlung in Stufe (c) bei einer Temperatur von weniger als 1000 und insbesondere weniger als 900°C durchführt.

7. Verfahren nach einem der Ansprüche 1 bis 6, **dadurch gekennzeichnet, daß** man das geformte Glaskeramik-Produkt gewünschter Geometrie mit einer Beschichtung versieht.

8. Verfahren nach Anspruch 7, **dadurch gekennzeichnet, daß** als Beschichtung eine Keramik, eine Sinterkeramik, eine Glaskeramik, ein Glas, eine Glasur und/oder ein Composit eingesetzt wird.

9. Verfahren nach einem der Ansprüche 1 bis 8, **dadurch gekennzeichnet, daß** die Schmelze des Ausgangsglases mindestens einen der folgenden weiteren Komponenten enthält:

| Komponente | Gew.-% |
|---|---|
| $ZrO_2$ | 0 bis 10,0 |
| $K_2O$ | 0 bis 13,5 |
| $P_2O_5$ | 0 bis 11,0 |
| Farb- und Fluoreszenzkomponenten | 0 bis 8,0 |
| Zusatzkomponenten | 0 bis 6,0 |

**10.** Verfahren nach einem der Ansprüche 1 bis 9, **dadurch gekennzeichnet, daß** man die Mengen der Komponenten unabhängig voneinander wie folgt wählt:

| Komponente | Gew.-% | |
|---|---|---|
| $SiO_2$ | 57,0 bis 75,0 | |
| $Al_2O_3$ | 0 bis 2,5 | |
| $La_2O_3$ | 0,1 bis 4,0 | |
| MgO | 0,1 bis 5,0 | |
| ZnO | 0 bis 6,0, | insbesondere 0,1 bis 5,0 |
| $ZrO_2$ | 0 bis 8,0, | insbesondere 0,1 bis 8,0 |
| $K_2O$ | 0 bis 9,0, | insbesondere 0,5 bis 7,0 |
| $Li_2O$ | 13,0 bis 19,0 | |
| $P_2O_5$ | 0 bis 8,0, | insbesondere 0,5 bis 8,0 |
| Farb- und Fluoreszenzkomponenten | 0,1 bis 8,0 | |
| Zusatzkomponenten | 0 bis 3,0. | |

**11.** Verfahren nach einem der Ansprüche 1 bis 10, **dadurch gekennzeichnet, daß** als Farb- oder Fluoreszenzkomponente mindestens eine der folgenden Verbindungen eingesetzt wird

| Komponente | Gew.-% |
|---|---|
| $CeO_2$ | 0,1 bis 5,0 |
| $V_2O_5$ | 0,01 bis 1,0 |
| $Fe_2O_3$ | 0,01 bis 1,0 |
| $MnO_2$ | 0,01 bis 3,0 |
| $TiO_2$ | 0,01 bis 5,0 |
| $Y_2O_3$ | 0,01 bis 2,0 |
| $Er_2O_3$ | 0,001 bis 2,0 |
| $Tb_2O_3$ | 0,001 bis 2,0 |
| $Eu_2O_3$ | 0,001 bis 2,0 |
| $Yb_2O_3$ | 0,001 bis 2,0 |
| $Gd_2O_3$ | 0,001 bis 2,0 |
| $Nd_2O_3$ | 0,001 bis 2,0 |
| $Pr_2O_3$ | 0,001 bis 2,0 |
| $Dy_2O_3$ | 0,001 bis 2,0 |
| $Ag_2O$ | 0,01 bis 2,0 |
| $SnO_2$ | 0,01 bis 3,0 |
| $Ta_2O_5$ | 0,001 bis 2,0. |

**12.** Verfahren nach einem der Ansprüche 1 bis 11, **dadurch gekennzeichnet, daß** die Zusatzkomponenten $B_2O_3$, $Na_2O$, BaO, F und/oder SrO sind.

**13.** Geformtes Glaskeramik-Produkt, welches nach dem Verfahren gemäß einem der Ansprüche 1 bis 12 erhältlich ist.

**14.** Glaskeramik-Produkt nach Anspruch 13, **dadurch gekennzeichnet, daß** es eine Säurebeständigkeit von kleiner

als 100 µg/cm$^2$ hat.

**15.** Glaskeramik-Produkt nach Anspruch 13 oder 14, **dadurch gekennzeichnet, daß** es einen CR-Wert von 0,05 bis 0,9, insbesondere 0,1 bis 0,75, aufweist.

**16.** Verwendung des geformten Glaskeramik-Produktes gemäß einem der Ansprüche 13 bis 15 als Dentalprodukt oder Bestandteil davon.

**17.** Geformtes Dentalprodukt, welches das Glaskeramik-Produkt gemäß einem der Ansprüche 13 bis 15 aufweist.

**18.** Geformtes Dentalprodukt nach Anspruch 17, **dadurch gekennzeichnet, daß** es ein Inlay, ein Onlay, eine Brücke, ein Stiftaufbau, eine Verblendung, ein Veneer, eine Facette, eine Krone oder eine Teilkrone ist.

**19.** Geformtes Dentalprodukt nach Anspruch 17, **dadurch gekennzeichnet, daß** es als Rohling vorliegt.

**Claims**

**1.** Process for the production of shaped translucent lithium disilicate glass-ceramic products by

(a) producing a melt of a starting glass which comprises the following components

| Component | wt.% |
|---|---|
| $SiO_2$ | 57.0 to 80.0 |
| $Al_2O_3$ | 0 to 5.0 |
| $La_2O_3$ | 0.1 to 6.0 |
| MgO | 0 to 5.0 |
| ZnO | 0 to 8.0 |
| $Li_2O$ | 11.0 to 19.0 |

where

(i) $Al_2O_3$ + $La_2O_3$ make up from 0.1 to 7.0 wt.% and
(ii) MgO + ZnO make up from 0.1 to 9.0 wt.%,

(b) shaping and cooling the melt of the starting glass in the desired manner, and

(c) subjecting the shaped glass product to at least one heat treatment in the temperature range from 400 to 1100°C in order to obtain a shaped glass-ceramic product in the form of a blank.

**2.** Process according to Claim 1, **characterised in that**

(d1)    the glass-ceramic product in the form of a blank is subjected to plastic deformation at a temperature of from 700 to 1200°C and through the application of pressure, in particular from 8 to 40 bar, to give a glass-ceramic product of the desired geometry.

**3.** Process according to Claim 1, **characterised in that**

(d2)    the glass-ceramic product in the form of a blank is converted into a glass-ceramic product of the desired geometry by machining.

**4.** Process according to Claim 3, **characterised in that** a blank which has undergone heat treatment at from 400 to 900°C is used in (d2).

**5.** Process according to Claim 3 or 4, **characterised in that** the shaped glass-ceramic product of the desired geometry obtained in step (d2) is subjected to at least one further heat treatment, in particular at from 700 to 900°C.

6. Process according to one of Claims 1 to 5, **characterised in that** the heat treatment in step (c) is carried out at a temperature of less than 1000 and in particular less than 900°C.

7. Process according to one of Claims 1 to 6, **characterised in that** the shaped glass-ceramic product of the desired geometry is provided with a coating.

8. Process according to Claim 7, **characterised in that** the coating employed is a ceramic, a sintered ceramic, a glass-ceramic, a glass, a glaze and/or a composite.

9. Process according to one of Claims 1 to 8, **characterised in that** the melt of the starting glass comprises at least one of the following further components:

| Component | wt.% |
|---|---|
| $ZrO_2$ | 0 to 10.0 |
| $K_2O$ | 0 to 13.5 |
| $P_2O_5$ | 0 to 11.0 |
| Colouring and fluorescent components | 0 to 8.0 |
| Additional components | 0 to 6.0 |

10. Process according to one of Claims 1 to 9, **characterised in that** the amounts of the components are selected, independently of one another, as follows:

| Component | wt.% | |
|---|---|---|
| $SiO_2$ | 57.0 to 75.0 | |
| $Al_2O_3$ | 0 to 2.5 | |
| $La_2O_3$ | 0.1 to 4.0 | |
| MgO | 0.1 to 5.0 | |
| ZnO | 0 to 6.0, | in particular 0.1 to 5.0 |
| $ZrO_2$ | 0 to 8.0, | in particular 0.1 to 8.0 |
| $K_2O$ | 0 to 9.0, | in particular 0.5 to 7.0 |
| $Li_2O$ | 13.0 to 19.0 | |
| $P_2O_5$ | 0 to 8.0, | in particular 0.5 to 8.0 |
| Colouring and fluorescent components | 0.1 to 8.0 | |
| Additional components | 0 to 3.0. | |

11. Process according to one of Claims 1 to 10, **characterised in that** the colouring or fluorescent component employed is at least one of the following compounds:

| Component | wt.% |
|---|---|
| $CeO_2$ | 0.1 to 5.0 |
| $V_2O_5$ | 0.01 to 1.0 |
| $Fe_2O_3$ | 0.01 to 1.0 |
| $MnO_2$ | 0.01 to 3.0 |
| $TiO_2$ | 0.01 to 5.0 |
| $Y_2O_3$ | 0.01 to 2.0 |
| $Er_2O_3$ | 0.001 to 2.0 |
| $Tb_2O_3$ | 0.001 to 2.0 |
| $Eu_2O_3$ | 0.001 to 2.0 |
| $Yb_2O_3$ | 0.001 to 2.0 |
| $Gd_2O_3$ | 0.001 to 2.0 |
| $Nd_2O_3$ | 0.001 to 2.0 |

(continued)

| Component | wt.% |
|---|---|
| $Pr_2O_3$ | 0.001 to 2.0 |
| $Dy_2O_3$ | 0.001 to 2.0 |
| $Ag_2O$ | 0.01 to 2.0 |
| $SnO_2$ | 0.01 to 3.0 |
| $Ta_2O_5$ | 0.001 to 2.0. |

12. Process according to one of Claims 1 to 11, **characterised in that** the additional components are $B_2O_3$, $Na_2O$, BaO, F and/or SrO.

13. Shaped glass-ceramic product which is obtainable by the process according to one of Claims 1 to 12.

14. Glass-ceramic product according to Claim 13, **characterised in that** it has an acid resistance of less than 100 µg/ $cm^2$.

15. Glass-ceramic product according to Claim 13 or 14, **characterised in that** it has a CR value of from 0.05 to 0.9, in particular from 0.1 to 0.75.

16. Use of the shaped glass-ceramic product according to one of Claims 13 to 15 as a dental product or a constituent thereof.

17. Shaped dental product which comprises the glass-ceramic product according to one of Claims 13 to 15.

18. Shaped dental product according to Claim 17, **characterised in that** it is an inlay, an onlay, a bridge, an abutment, a facing, a veneer, a facet, a crown or a partial crown.

19. Shaped dental product according to Claim 17, **characterised in that** it is in the form of a blank.


**Revendications**

1. Procédé pour la fabrication de produits translucides mis en forme en vitrocéramique à base de disilicate de lithium, dans lequel :

   (a) on produit une masse fondue d'un verre de départ qui contient les composants suivants

   | Composant | % en poids |
   |---|---|
   | $SiO_2$ | 57,0 à 80,0 |
   | $Al_2O_3$ | 0 à 5,0 |
   | $La_2O_3$ | 0,1 à 6,0 |
   | MgO | 0 à 5,0 |
   | ZnO | 0 à 8,0 |
   | $Li_2O$ | 11,0 à 19,0 |

   (i) $Al_2O_3$ + $La_2O_3$ constituant de 0,1 à 7,0 % en poids et
   (ii) MgO + ZnO constituant de 0,1 à 9,0 % en poids,

   (b) on met en forme et on refroidit de la façon désirée de la masse fondue du verre de départ, et
   (c) on soumet le produit en verre mis en forme à au moins un traitement thermique dans la plage de températures allant de 400 à 1 100°C, pour obtenir un produit vitrocéramique mis en forme, présent en tant qu'ébauche.

2. Procédé selon la revendication 1, **caractérisé en ce que**

(d1)    on soumet à une déformation plastique le produit vitrocéramique présent en tant qu'ébauche, à une température de 700 à 1 200°C et par application de pression, en particulier de 8 à 40 bars, pour obtenir un produit vitrocéramique de géométrie désirée.

**3.**   Procédé selon la revendication 1, **caractérisé en ce que**

(d2)    on transforme le produit vitrocéramique présent sous forme d'ébauche, par usinage à enlèvement de copeaux, en un produit vitrocéramique de géométrie désirée.

**4.**   Procédé selon la revendication 3, **caractérisé en ce qu'**on utilise en (d2) une ébauche qui a été soumise à un traitement thermique à 400-900°C.

**5.**   Procédé selon la revendication 3 ou 4, **caractérisé en ce qu'**on soumet le produit vitrocéramique mis en forme, de géométrie désirée, obtenu dans l'étape (d2), à au moins un autre traitement thermique, en particulier à 700-900°C.

**6.**   Procédé selon l'une quelconque des revendications 1 à 5, **caractérisé en ce qu'**on effectue le traitement thermique dans l'étape (c) à une température de moins de 1 000 et en particulier de moins de 900°C.

**7.**   Procédé selon l'une quelconque des revendications 1 à 6, **caractérisé en ce qu'**on munit d'un revêtement le produit vitrocéramique mis en forme, de géométrie désirée.

**8.**   Procédé selon la revendication 7, **caractérisé en ce qu'**on utilise comme revêtement une céramique, une céramique frittée, une vitrocéramique, un verre, une glaçure et/ou un composite.

**9.**   Procédé selon l'une quelconque des revendications 1 à 8, **caractérisé en ce que** la masse fondue du verre de départ contient au moins l'un des autres composants suivants :

| Composant | % en poids |
|---|---|
| $ZrO_2$ | 0 à 10,0 |
| $K_2O$ | 0 à 13,5 |
| $P_2O_5$ | 0 à 11,0 |
| Composants colorants et fluorescents | 0 à 8,0 |
| Composants supplémentaires | 0 à 6,0 |

**10.**  Procédé selon l'une quelconque des revendications à 9, **caractérisé en ce qu'**on choisit les quantités des composants, indépendamment les uns des autres, comme suit :

| Composant | % en poids | |
|---|---|---|
| $SiO_2$ | 57,0 à 75,0 | |
| $Al_2O_3$ | 0 à 2,5 | |
| $La_2O_3$ | 0,1 à 4,0 | |
| MgO | 0,1 à 5,0 | |
| ZnO | 0 à 6,0, | en particulier 0,1 à 5,0 |
| $ZrO_2$ | 0 à 8,0, | en particulier 0,1 à 8,0 |
| $K_2O$ | 0 à 9,0, | en particulier 0,5 à 7,0 |
| $Li_2O$ | 13,0 à 19,0 | |
| $P_2O_5$ | 0 à 8,0, | en particulier 0,5 à 8,0 |
| Composants colorants et fluorescents | 0,1 à 8,0 | |
| Composants supplémentaires | 0 à 3,0. | |

**11.**  Procédé selon l'une quelconque des revendications 1 à 10, **caractérisé en ce qu'**on utilise comme composant colorant ou fluorescent au moins l'un des composés suivants

| Composant | % en poids |
|---|---|
| $CeO_2$ | 0,1 à 5,0 |
| $V_2O_5$ | 0,01 à 1,0 |
| $Fe_2O_3$ | 0,01 à 1,0 |
| $MnO_2$ | 0,01 à 3,0 |
| $TiO_2$ | 0,01 à 5,0 |
| $Y_2O_3$ | 0,01 à 2,0 |
| $Er_2O_3$ | 0,001 à 2,0 |
| $Tb_2O_3$ | 0,001 à 2,0 |
| $Eu_2O_3$ | 0,001 à 2,0 |
| $Yb_2O_3$ | 0,001 à 2,0 |
| $Gd_2O_3$ | 0,001 à 2,0 |
| $Nd_2O_3$ | 0,001 à 2,0 |
| $Pr_2O_3$ | 0,001 à 2,0 |
| $Dy_2O_3$ | 0,001 à 2,0 |
| $Ag_2O_3$ | 0,01 à 2,0 |
| $SnO_2$ | 0,01 à 3,0 |
| $Ta_2O_5$ | 0,001 à 2,0. |

**12.** Procédé selon l'une quelconque des revendications 1 à 11, **caractérisé en ce que** les composants supplémentaires sont $B_2O_3$, $Na_2O$, BaO, F et/ou SrO.

**13.** Produit vitrocéramique mis en forme susceptible d'être obtenu par le procédé selon l'une quelconque des revendications 1 à 12.

**14.** Produit vitrocéramique selon la revendication 13, **caractérisé en ce qu'**il a une résistance à l'acide de moins de 100 $\mu g/cm^2$.

**15.** Produit vitrocéramique selon la revendication 13 ou 14, **caractérisé en ce qu'**il présente un indice CR de 0,05 à 0,9, en particulier de 0,1 à 0,75.

**16.** Utilisation du produit vitrocéramique mis en forme selon l'une quelconque des revendications 13 à 15, en tant que produit dentaire ou composant d'un tel produit.

**17.** Produit dentaire mis en forme, qui comporte le produit vitrocéramique selon l'une quelconque des revendications 13 à 15.

**18.** Produit dentaire mis en forme selon la revendication 17, **caractérisé en ce qu'**il s'agit d'un inlay, d'un onlay, d'un bridge, d'un pivot, d'un revêtement, d'un placage, d'une facette, d'une couronne ou d'une couronne partielle.

**19.** Produit dentaire mis en forme selon la revendication 17, **caractérisé en ce qu'**il se trouve sous forme d'ébauche.